# EUROPÄISCHE PATENTSCHRIFT

(11) **EP 0 926 131 B1**
(45) Veröffentlichungstag und Bekanntmachung des Hinweises auf die Patenterteilung: **11.02.2004**
(21) Anmeldenummer: 98122293.8
(22) Anmeldetag: 24.11.1998
(51) Int. Cl.: C07C 213/00, C07C 215/42, C07C 213/02, C07C 213/10, C07D 473/32, C12P 41/00, C12P 13/00, C07D 473/00

(54) **Verfahren zur Herstellung von 1-Amino-4-(hydroxymethyl)-2-cyclopenten**
Process for the preparation of 1-amino-4-(hydroxymethyl)-2-cyclopentene
Procédé pour la préparation de 1-amino-4-(hydroxymethyl)-2-cyclopenten

(30) Priorität: 27.11.1997 CH 273997; 03.12.1997 CH 278197; 21.01.1998 CH 13398; 27.03.1998 CH 72398; 07.10.1998 EP 98118895
(43) Veröffentlichungstag der Anmeldung: 30.06.1999
(73) Patentinhaber: Lonza AG, 3930 Visp (CH)
(72) Erfinder: Brieden, Walter Dr., 3902 Brig (CH); Schröer, Josef Dr., 3952 Susten (CH); Bernegger-Egli, Christine Dr., 3985 Münster (CH); Urban, Eva Maria, 3930 Visp (CH); Petersen, Michael Dr., 3930 Visp (CH); Roduit, Jean-Paul Dr., 3979 Grone (CH); Berchtold, Katja, 3937 Baltschieder (CH); Breitbach, Holger Dr., 3937 Baltschieder (CH)
(74) Vertreter: Winter, Brandl & Partner

(56) Entgegenhaltungen:
- EP-A- 0 434 450
- EP-A- 0 878 548
- WO-A-93/17020
- WO-A-95/21161
- WO-A-97/45529
- CAMPBELL, JEFFREY A. ET AL: "Chirospecific Syntheses of Precursors of Cyclopentane and Cyclopentene Carbocyclic Nucleosides by [3 + 3]-Coupling and Transannular Alkylation" J. ORG. CHEM. (1995), 60(14), 4602-16 CODEN: JOCEAH;ISSN: 0022-3263, XP002041314
- N. KATAGIRI ET AL.: "Deamination of 9-(hydroxymethylated cycloalkyl)-9H-adenines (carbocyclic adenine nucleosides) by adenosine deaminase: Effect of the high-pressure upon deamination rate and enantioselectivity" NUCLEOSIDES & NUCLEOTIDES., Bd. 15, Nr. 1-3, 1996, Seiten 631-647, XP002124310 MARCEL DEKKER, INC., US ISSN: 0732-8311
- J.R. MALPASS ET AL.: "Reaction of Chlorosuphonyl Isocyanate with 1,3-Dienes. Control of 1,2- and 1,4-Addition Pathways and the Synthesis of Aza- and Oxa-bicyclic Systems" JOURNAL OF THE CHEMICAL SOCIETY, PERKIN TRANSACTIONS 1,1977, Seiten 874-884, XP002104986 LETCHWORTH GB

## Beschreibung

Die vorliegende Erfindung betrifft ein Verfahren zur Herstellung eines Aminoalkohols der Formel 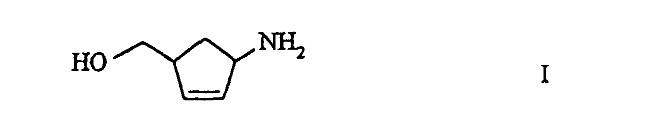 in Form des Racemats oder eines seiner optisch aktiven Isomeren, nämlich (1R,4S)- oder (1S,4R)-1-Amino-4-(hydroxymethyl)-2-cyclopenten der Formeln 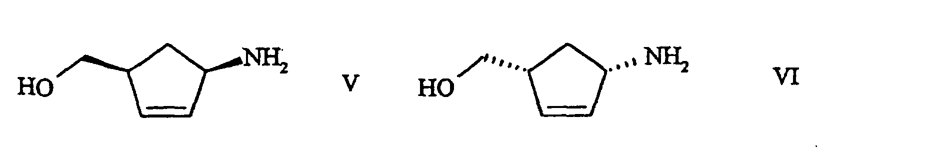 oder Salzen davon.

(1R,4S)-1-Amino-4-(hydroxymethyl)-2-cyclopenten der Formel V ist ein wichtiges Zwischenprodukt zur Herstellung von carbocyclischen Nukleosiden wie z. B. Carbovir® (Campbell et al., J. Org. Chem. 1995, 60, 4602 - 4616).

Ein Verfahren zur Herstellung von (1R,4S)-1-Amino-4-(hydroxymethyl)-2-cyclopenten ist beispielsweise von Campbell et al. (ibid) und von Park K. H. & Rapoport H. (J. Org. Chem. 1994, 59, 394 - 399) beschrieben. Bei diesen Verfahren dient als Edukt entweder D-glucon-δ-lacton oder D-Serin, wobei ca. 15 Synthesestufen bis zur Bildung von (1R,4S)-N-tert-Butoxycarbonyl-4-hydroxymethyl-2-cyclopenten, welches dann zum (1R,4S)-1-Amino-4-(hydroxymethyl)-2-cyclopenten deprotektioniert wird, notwendig sind. Diese beiden Verfahren sind kostspielig, umständlich und grosstechnisch nicht gangbar.

Die WO 93/17020 beschreibt ein Verfahren zur Herstellung von (1R,4S)-1-Amino-4-(hydroxymethyl)-2-cyclopenten, wobei (1R,4S)-4-Amino-2-cyclopenten-1-carbonsäure mit Lithiumaluminiumhydrid zum gewünschten Produkt reduziert wird. Nachteilig bei diesem Verfahren ist zum einen, dass auch die Doppelbindung des Cyclopentenringes reduziert wird, die schlechte Handhabbarkeit von Lithiumaluminiumhydrid und zum anderen, dass es zu kostspielig ist.

Taylor S. J. et al. (Tetrahetron: Asymmetry Vol. 4, No. 6, 1993, 1117 - 1128) beschreiben ein Verfahren zur Herstellung von (1R,4S)-1-Amino-4-(hydroxymethyl)-2-cyclopenten ausgehend von (±)-2-Azabicyclo[2.2.1]hept-5-en-3-on als Edukt. Dabei wird das Edukt mittels Mikroorganismen der Spezies Pseudomonas solanacearum oder Pseudomonas fluorescens in (1R,4S)-2-Azabicyclo[2.2.1]hept-5-en-3-on überführt, welches dann mit Di-tert-butyldicarbonat in das (1R,4S)-N-tert-butoxycarbonyl-2-azabicyclo[2.2.1]hept-5-en-3-on umgesetzt wird. Letzteres wird mit Natriumborhydrid und Trifluoressigsäure zum gewünschten Produkt reduziert. Auch ist dieses Verfahren viel zu kostspielig.

Desweiteren beschreiben Martinez et al. (J. Org. Chem. 1996, 61, 7963 - 7966) eine 10stufige Synthese von (1R,4S)-1-Amino-4-(hydroxymethyl)-2-cyclopenten ausgehend von Dialkylmalonsäurediethylester. Auch dieses Verfahren hat den Nachteil, dass es umständlich und grosstechnisch nicht gangbar ist.

Bekannt ist auch, dass man N-substituierte-(±)-2-Azabicyclo[2.2.1]hept-5-en-3-one, welche einen elektronenziehenden Substituenten tragen, mit einem Metallhydrid zu den entsprechenden N-substituierten Aminoalkoholen reduzieren kann (Katagiri et al., Tetrahedron Letters, 1989, 30, 1645 - 1648; Taylor et al., ibid). Katagiri et al. (Nucleosides & Nucleotides 1996, 15(1-3), 631-647) beschreiben eine dreistufige Synthese von 1-Amino-4-hydroxymethyl-2-cyclopenten aus 2-Azabicyclo[2.2.1]hept-5-en-3-on. Dabei wird im ersten Schritt die Amidbindung von 2-Azabicyclo[2.2.1]hept-5-en-3-on gegenüber einer reduktiven Öffnung durch Einführung einer tert-Butyloxycarbonylgruppe am Stickstoff aktiviert, im zweiten Schritt wird die aktivierte Amidbindung mittels Natriumborhydrid reduktiv unter Ausbildung von N-(tert-Butyloxycarbonyl)-1-amino-4-hydroxymethyl-2-cyclopenten geöffnet, und im dritten Schritt wird diese tert-Butyloxycarbonylgruppe unter Ausbildung von 1-Amino-4-hydroxymethyl-2-cyclopenten abgespalten.

Im Gegensatz dazu ist bekannt, dass unsubstituiertes (±)-2-Azabicyclo[2.2.1]hept-5-en-3-on der Formel 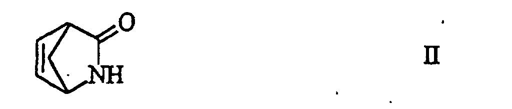 mit Lithiumaluminiumhydrid zu (±)-2-Azabicyclo[2.2.1]octen reduziert wird (Malpass & Tweedle, J. Chem. Soc., Perkin Trans 1, 1977, 874 - 884) und dass die direkte Reduktion von (±)-2-Azabicyclo[2.2.2]hept-5-en-3-on zum entsprechenden Aminoalkohol bislang als nicht möglich galt (Katagiri et al., ibid; Taylor et al., ibid).

Bekannt ist auch, racemisches 1-Amino-4-(hydroxymethyl)-2-cyclopenten mittels (-)- Dibenzoylweinsäure zu spalten (US-A 5 034 394). Diese Umsetzung hat einerseits den Nachteil, dass die (-)-Dibenzoylweinsäure teuer ist, anderseits, dass die Trennung in Gegenwart eines genau definierten Gemisches von Acetonitril und Ethanol erfolgen muss. Dieses Lösungsmittelgemisch kann nicht getrennt werden und muss der Verbrennung zugeführt werden.

Aufgabe der vorliegenden Erfindung war es, ein einfaches, ökonomisches und kostengünstiges Verfahren zur Herstellung von (1R,4S)-1-Amino-4-(hydroxymethyl)-2-cyclopenten zur Verfügung zu stellen.

Überraschenderweise wurde nun gefunden, dass wenn man (±)-2-Azabicyclo[2.2.1]hept-5-en-3-on der Formel 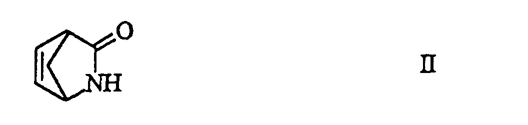 in Form des Racemats oder eines seiner optisch aktiven Isomere, mit einem Metallhydrid reduziert, der Aminoalkohol der Formel 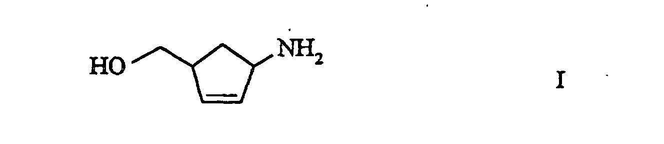 in Form des Racemats oder eines seiner optisch aktiven Isomere, auf einfache Weise erhalten wird. Vorzugsweise wird der racemische cis-Aminoalkohol der Formel I erhalten.

Wie fachmännisch üblich kann der Aminoalkohol der Formel I mit einer Säure in die entsprechenden Salze überführt werden, wie beispielsweise in Hydrohalogenidsalze. Als Hydrohalogenidsalze sind Hydrobromide und Hydrochloride geeignet.

Das Edukt, das (±)-2-Azabicyclo[2.2.1]hept-5-en-3-on, kann gemäss der EP-A 0 508 352 hergestellt werden.

Als Metallhydride können Alkalimetall- oder Erdalkalimetallhydride sowie binäre oder komplexe Metallhydride der Bor- oder Aluminiumgruppe eingesetzt werden, beispielsweise Alkalimetall- und Erdalkalimetallborhydride und Alkalimetall- und Erdalkalimetallaluminiumhydride.

Als Alkalimetall- oder Erdalkalimetallhydride sind LiH, NaH, KH, BeH₂, MgH₂ oder CaH₂ geeignet. Als binäre Alkalimetall- oder Erdalkalimetallborhydride können NaBH₄, LiBH₄, KBH₄, NaAlH₄, LiAlH₄, KAlH₄, Mg(BH₄)₂, Ca(BH₄)₂, Mg(AlH₄)₂, Ca(AlH₄)₂ verwendet werden. Komplexe Metallhydride der Bor- oder Aluminiumgruppe können die allgemeine Formel M¹ M²HₙLₘ haben, worin n eine ganze Zahl von 1 bis 4 ist und m eine ganze Zahl von 4 bis 4 minus der entsprechenden Zahl n ist, M¹ ein Alkalimetallatom bedeutet, M² Bor oder Aluminium bedeutet und L C₁₋₄-Alkyl, C₁₋₄-Alkenyl, C₁₋₄-Alkoxy, CN oder ein Amin ist oder die komplexen Metallhydride können die allgemeine Formel M²H_{O}Lₚ haben, worin M² die genannte Bedeutung hat und O eine ganze Zahl von 0 bis 3 ist und p eine ganze Zahl von 3 bis 3 minus der entsprechenden Zahl p ist. Als M¹ M² HₙLₘ können LiBH (C₂H₅)₃, LiBHₓ (OCH₃)₄₋ₓ, worin x eine ganze Zahl von 1 bis 3 bedeutet, LiAlH(OC(CH₃)₃)₃, NaAlH₂(OC₂H₄OCH₃)₂, NaAlH₂(C₂H₅)₂ oder NaBH₃CN eingesetzt werden. Vorzugsweise wird die Reduktion mit einem Metallborhydrid durchgeführt.
Wie fachmännisch bekannt, können die erwähnten Metallhydride wie z. B. LiBH₄ auch "in situ" erzeugt werden. Gängige Darstellungsmethoden für LiBH₄ sind beispielsweise die Umsetzung eines Alkalimetallborhydrids mit einem Lithiumhalogenid (H. C. Brown et al., Inorg. Chem. 20, 1981, 4456 - 4457), die Umsetzung von LiH mit B₂O₃ in Gegenwart von Wasserstoff und einem Hydrierkatalysator (EP-A 0 512 895), die Umsetzung von LiH mit (H₅C₂)OBF₃ (DE-OS 94 77 02) und die von LiH mit B(OCH₃)₃ (US-A 2,534,533).

Zweckmässig werden die Metallhydride in einem molaren Verhältnis von 1 bis 5 pro mol (±)-2-Azabicyclo[2.2.1]hept-5-en-3-on eingesetzt.

Vorzugsweise werden die Metallhydride, insbesondere NaBH₄, mit Lithiumsalzzusätzen verwendet. Als Lithiumsalze können LiCl, LiBr, LiF, LiJ, Li₂SO₄, LiHSO₄, Li₂CO₃, Li(OCH₃) und LiCO₃ verwendet werden.

Zweckmässig wird die Reduktion unter Inertgasatmosphäre, beispielsweise unter Argon- oder Stickstoffatmosphäre, durchgeführt.

Die Reduktion kann bei einer Temperatur von -20 bis 200 °C, vorzugsweise bei einer Temperatur von 60 bis 150 °C, durchgeführt werden.

Als Lösungsmittel sind aprotische oder protische organische Lösungsmittel geeignet. Als aprotische organische Lösungsmittel können Ether oder Glykolether wie beispielsweise Diethylether, Dibutylether, Ethylmethylether, Diisopropylether, tert-Butylmethylether, Anisol, Dioxan, Tetrahydrofuran, Mono-Glyme, Diglyme und Formaldehyddimethylacetal eingesetzt werden. Als protische organische Lösungsmittel sind C₁₋₆-Alkohole wie Methanol, Ethanol, Propanol, Isopropanol, Butanol, tert-Butanol, Pentanol, tert-Amylalkohol oder Hexanol sowie Mischungen von diesen mit Wasser geeignet. Als protische organische Lösungsmittel ebenso geeignet sind Mischungen von einem der genannten Ether, Glykolether mit Wasser oder mit einem der genannten Alkohole wie eine Mischung von einem C₁₋₆-Alkohol mit einem Ether oder Glykolether, insbesondere eine Mischung von Methanol, Ethanol oder Wasser mit Diethylether, Tetrahydrofuran, Dioxan, Glyme oder Diglyme. Vorzugsweise wird als Lösungsmittel ein protisches organisches Lösungsmittel, wie eine Mischung von einem C₁₋₆-Alkohol oder Wasser mit einem Ether oder Glykolether, eingesetzt.

In einer bevorzugten Ausführungsform wird die Reduktion in Gegenwart eines Zusatzes, beispielsweise in Gegenwart von Wasser oder eines ein- oder mehrwertigen C₁₋₆-Alkohols durchgeführt. Als einwertige C₁₋₆-Alkohol können Methanol, Ethanol, Methoxyethanol, n-Propanol, Isopropanol, Isobutanol, tert-Butanol, n-Butanol verwendet werden. Als mehrwertige Alkohole können bspw. Diole wie Butandiol und Triole wie Glycerin verwendet werden. Insbesondere wird als C₁₋₆-Alkohol Methanol oder Ethanol verwendet. Zweckmässig wird dabei der C₁₋₆-Alkohol in einem molaren Verhältnis von 2 bis 15 pro mol (±)-2-Azabicyclo[2.2.1]hept-5-en-3-on eingesetzt.

Wird die Umsetzung in Gegenwart der genannten Alkohole durchgeführt, kann in situ (intermediär) der entsprechende Aminosäureester gebildet werden. D. h. wird als Edukt (+)-2-Azabicyclo[2.2.1]hept-5-en-3-on verwendet, kann erfindungsgemäss der entsprechende (+)-Aminosäureester gebildet werden.

Wird als Edukt (-)-2-Azabicyclo[2.2.1]hept-5-en-3-on verwendet, kann erfindungsgemäss dementsprechend der (-)-Aminosäureester intermediär gebildet werden.

### Beispiele:

### Beispiel 1

### Reduktion von 2-Azabicyclo[2.2.1]hept-5-en-3-on

### 1.1 Herstellung von cis-(±)-1-Amino-4-(hydroxymethyl)-2-cyclopenten

Eine Suspension von (±)-2-Azabicyclo[2.2.1]hept-5-en-3-on (10,00 g, 91,6 mmol) und Lithiumborhydrid (4,00 g, 183,7 mmol) in trockenem Dioxan (100 ml) wurde unter Inertgasatmosphäre (Argon) 4 h bei 110 °C unter Rückflusstemperatur erhitzt. Nach dieser Zeit waren ca. 20 - 25 % des Eduktes zum Produkt umgesetzt (GC-Analyse mit internem Standard Benzophenon nach Aufarbeitung des Reaktionsgemisches; Aufarbeitung: 0,05 ml des Reaktionsgemisches wurden mit 0,1 ml 1M HCl gequencht und direkt im Anschluss mit 0,2 ml 1M NaOH basisch gestellt).

Der Strukturnachweis des Produktes erfolgte durch H-NMR, GC und GC-MS.

### 1.2 Herstellung von cis-(+)-1-Amino-4-(hydroxymethyl)-2-cyclopenten

In einem 25 ml Rundkolben wurden 1,0 g (9,2 mmol) (+)-2-Azabicyclo[2.2.1]hept-5-en-3-on und 0,4 g (18,4 mmol) Lithiumborhydrid, unter Inertgasatmosphäre, in 10 ml Dioxan vorgelegt und 3 h bei 110 °C refluxiert. Überschüssiges Reduktionsmittel wurde durch Zugabe von ca. 5 ml halbkonzentrierter HCl vernichtet (auf pH 3 gestellt). Sofort im Anschluss daran wurde durch Zugabe von ca. 1 ml gesättigter NaHCO₃-Lösung bei pH 8 gepuffert. GC Analyse zeigte hier die Bildung des Produktes. Das gesamte Reaktionsgemisch wurde nun zur Trockene eingedampft und mittels Säulenchromatographie (Gradient: Hexan/Essigester/MeOH = 1:1:1 -> MeOH) gereinigt. Auf diese Weise wurde cis-(+)-2-Azabicyclo[2.2.1]hept-5-en-3-on reisoliert und der entsprechende (+)-Aminoalkohol gewonnen.

### 1.3 Herstellung von cis-(-)-1-Amino-4-(hydroxymethyl)-2-cyclopenten

In einem 25 ml Rundkolben wurden 1,0 g (9,2 mmol) (-)-2-Azabicyclo[2.2.1]hept-5-en-3-on und 0,4 g (18,4 mmol) Lithiumborhydrid, unter Inertgasatmosphäre, in 10 ml Dioxan vorgelegt und 3 h bei 110 °C refluxiert. Überschüssiges Reduktionsmittel wurde durch Zugabe von ca. 5 ml halbkonzentrierter HCl vernichtet (auf pH 3 gestellt). Sofort im Anschluss daran wurde durch Zugabe von ca. 1 ml gesättigter NaHCO₃-Lösung bei pH 8 gepufferet. GC Analyse zeigte hier die Bildung des Produktes in 18 % Ausbeute an. (GC-Standard ist Benzophenon). Das gesamte Reaktionsgemisch wurde nun zur Trockene eingedampft und mittels Säulenchromatographie (Gradient: Hexan/Essigester/MeOH = 1:1:1 -> MeOH) gereinigt. Auf diese Weise wurde 0,43g (43 %) cis-(-)-2-Azabicyclo[2.2.1]hept-5-en-3-on reisoliert sowie 0,04g (4 %) entsprechender (-)-Aminoalkohol gewonnen.
Durch HPLC war nur das (-)-Enantiomer des Aminoalkohols detektierbar. Der ee des Produktes ist somit >98 %.

### 1.4 Herstellung von cis-(±)-1-Amino-4-(hydroaymethyl)-2-cyclopenten in einem Alkohol

In einem 100 ml Rundkolben mit magnetischer Rührung wurden 3,0 g (27,5 mmol) (±)-2-Azabicyclo[2.2.1]hept-5-en-3-on und 1,2 g (28,3 mmol) Lithiumborhydrid, unter Inertgasatmosphäre, in 35 g 2-Butanol vorgelegt und 3 h bei 60 °C gerührt.
GC-Analyse eines Musters (Aufarbeitung: 0,1 g Probe mit 0,2 ml 1M HCl sauergestellt, dann mit 0,1 ml gesättigter NaHCO₃ rasch basisch gestellt) zeigte nach dieser Zeit die Bildung des Produktes in 12 %iger Ausbeute an (GC-Standard ist Benzophenon).

### 1.5 Herstellung von cis-(±)-1-Amino-4-(hydroxymethyl)-2-cyclopenten in einem Alkohol/Ether-Gemisch

In einem 10 ml Rundkolben wurden unter Inertgasatmosphäre 0,5 g (4,6 mmol) (±)-2-Azabicyclo[2.2.1]hept-5-en-3-on und 0,59 g (18,4 mmol) Methanol in 7,5 ml Dioxan (abs.) vorgelegt. Dazu gab man 0,21 g (9,2 mmol) Lithiumborhydrid und heizte für 4 h bei 60°C. Dann kühlte man mit einem Eis/Wasser Bad auf 5°C und gab vorsichtig ca. 10 ml halbkonzentrierte HCl zum Reaktionsgemisch (heftige Reaktion, Gasentwicklung), wodurch eine gelblich klare Lösung entstand. Diese Lösung wurde direkt durch ein quantitatives ionenchromatografisches Verfahren analysiert. Sie enthielt 0,60 mmol (13,1%) cis-(±)-2-Azabicyclo[2.2.1]hept-5-en-3-on (bestimmt als HCl-Salz der entsprechenden Aminosäure, welche das saure Hydrolyseprodukt des (±)-2-Azabicyclo[2.2.1]hept-5-en-3-ons ist) und 3,06 mmol Produkt, entsprechend einer Ausbeute von 66,8%, Aminoalkohol.

### 1.6 Herstellung von cis-(±)-1-Amino-4-(hydroxymethyl)-2-cyclopenten in Gegenwart von Zusätzen wie Wasser oder verschiedenen Alkoholen

In einem 10 ml Rundkolben wurden 0,50 g (4,6 mmol) (±)-2-Azabicyclo[2.2.1]hept-5-en-3-on und 0,30 g (13,7 mmol) Lithiumborhydrid in 7,5 ml abs. Dioxan vorgelegt und auf 60 °C aufgeheizt. Bei dieser Temperatur wurde während 30 min. mittels Spritze X mmol des Zusatzes Y (Alkohol oder Wasser) zugetropft. Nun wurde 2 h bei 60 °C gerührt, abgekühlt auf ca. 20 °C und auf ca. 10 ml halbkonzentrierte HCl gegeben. Der Gehalt wurde dann direkt durch ein quantitatives ionenchromatografisches Verfahren bestimmt (vgl. Tabelle 1).

**Tabelle 1**

| Beispiel | Zusatz Y | X | X | (±)-2-Azabicyclo[2.2.1]-hept-5-en-3-on | Aminoalkohol |
|---|---|---|---|---|---|
| | | mmol | Aequivalente | Ausbeute % | |
| 1.6.1 | - | - | - | 15 | 52 |
| 1.6.2 | Wasser | 17,1 | 1,25 | 23,3 | 67,5 |
| 1.6.3 | Wasser | 34,3 | 2,5 | 32,3 | 58,3 |
| 1.6.4 | Methanol | 34,3 | 2,5 | 4,5 | 83,1 |
| 1.6.5 | Ethanol | 34,3 | 2,5 | 6,5 | 74,7 |
| 1.6.6 | Isopropanol | 34,3 | 2,5 | 28,1 | 52,3 |

### 1.7 Herstellung von cis-(±)-1-Amino-4-(hydroxymethyl)-2-cyclopenten mit verschiedenen Methanol-Mengen

Die Reaktion wurde unter den gleichen Bedingungen wie Beispiel 1.6 durchgeführt, jedoch mit der Ausnahme, dass anstatt des Zusatzes Y die Reaktion in verschiedenen Methanol-Konzentrationen durchgeführt wurde. Die Ergebnisse sind in Tabelle 2 zusammengefasst.

**Tabelle 2**

| Beispiel | Methanol | Methanol | (±)-2-Azabicyclo-[2.2.1] hept-5-en-3-on | Aminoalkohol |
|---|---|---|---|---|
| | mmol | Aequivalente | Ausbeute % | |
| 1.7.1 | 9,2 | 1 | 27,5 | 44,8 |
| 1.7.2 | 18,3 | 2 | 13,1 | 66,8 |
| 1.7.3 | 27,5 | 3 | 24,7 | 54,8 |
| 1.7.4 | 36,6 | 4 | 5,7 | 56,8 |
| 1.7.5 | 45,8 | | 12,0 | 58,3 |
| 1.7.6 | 55,0 | 6 | 7,2 | 33,0 |

### 1.8 Herstellung von cis-(±)-1-Amino-4-(hydroxymethyl)-2-cyclopenten mit verschiedenen Lösungsmitteln

Die Reaktion wurde unter den gleichen Bedingungen wie Beispiel 1.6 durchgeführt, jedoch mit der Ausnahme, dass anstatt des Zusatzes Y 1,1 g Methanol zugetropft wurden und anstatt Dioxan als Lösungsmittel die Reaktion in verschiedenen Lösungsmitteln (7,5 ml) durchgeführt wurde und der Gehalt bestimmt wurde. Die Ergebnisse sind in Tabelle 3 zusammengefasst.

**Tabelle 3**

| Beispiel | Lösungsmittel X | (±)-2-Azabicyclo[2.2.1] hept-5-en-3-on | Aminoalkohol |
|---|---|---|---|
| | | Ausbeute % | |
| 1.8.1 | Dioxan | 13,6 | 79,8 |
| 1.8.2 | Diethylether | 10,8 | 68,6 |
| 1.8.3 | Tetrahydrofuran | 22,4 | 67,6 |
| 1.8.4 | Diisopropyl-Ether | 12,6 | 51,3 |
| 1.8.5 | tert-Butylmethyl-Ether | 10,0 | 71,3 |
| 1.8.6 | Mono-Glyme | 15,5 | 75,3 |
| 1.8.7 | Formaldehyddimethylacetal | 12,0 | 74,2 |

### 1.9 Herstellung von cis-(±)-1-Amino-4-(hydroxymethyl)-2-cyclopenten mit verschiedenen LiBH₄-Zugaben

Die Reaktion wurde unter den gleichen Bedingungen wie in Beispiel 1.6 durchgeführt, jedoch mit der Ausnahme, dass anstatt Zusatz Y 2,5 mol Methanol eingesetzt wurden und die Reaktion mit verschiedenen LiBH₄-Konzentrationen durchgeführt und der Gehalt bestimmt wurde. Die Ergebnisse sind in Tabelle 4 zusammengefasst.

**Tabelle 4**

| Beispiel | LiBH₄ | LiBH₄ | (±)-2-Azabicyclo[2.2.1] hept-5-en-3-on | Aminoalkohol |
|---|---|---|---|---|
| | mmol | Aequivalente | Ausbeute % | |
| 1.9.1 | 4,6 | 1 | 11,9 | 47,9 |
| 1.9.2 | 6,9 | 1,5 | 9,6 | 45,6 |
| 1.9.3 | 9,2 | 2 | 12,7 | 71,3 |
| 1.9.4 | 11,5 | 2,5 | 13,3 | 74,5 |
| 1.9.5 | 13,8 | 3 | 12,8 | 77,1 |
| 1.9.6 | 16,1 | 3,5 | 12,7 | 62,4 |

### 1.10 Herstellung von cis-(+)- oder (-)-1-Amino-4-(hydroxymethyl)-2-cyclopenten in Gegenwart verschiedener Akohole bzw. in Gegenwart von Wasser in verschiedenen Lösungsmitteln

In einem 10 ml Rundkolben mit magnetischer Rührung wurden 0,50 g (4,6 mmol) (+)-oder (-)-2-Azabicyclo[2.2.1]hept-5-en-3-on und 0,30 g (13,7 mmol) Lithiumborhydrid in 6 ml verschiedener Lösungsmittel vorgelegt und auf 60°C aufgeheizt. Bei dieser Temperatur wird während 30 min. mittels Spritze 34,3 mmol des Zusatzes Y zugetropft. Nun wurde 2h bei 60°C gerührt, abgekühlt auf ca. 20°C und auf ca. 10 ml halbkonzentrierte HCl gegeben.

Der Gehalt wird dann direkt durch ein quantitatives ionenchromatografisches Verfahren bestimmt (vgl. Tabelle 5). Der ee-Wert des Produktes wurde mittels HPLC ermittelt.
Die Ergebnisse sind in Tabelle 5 zusammengefasst.

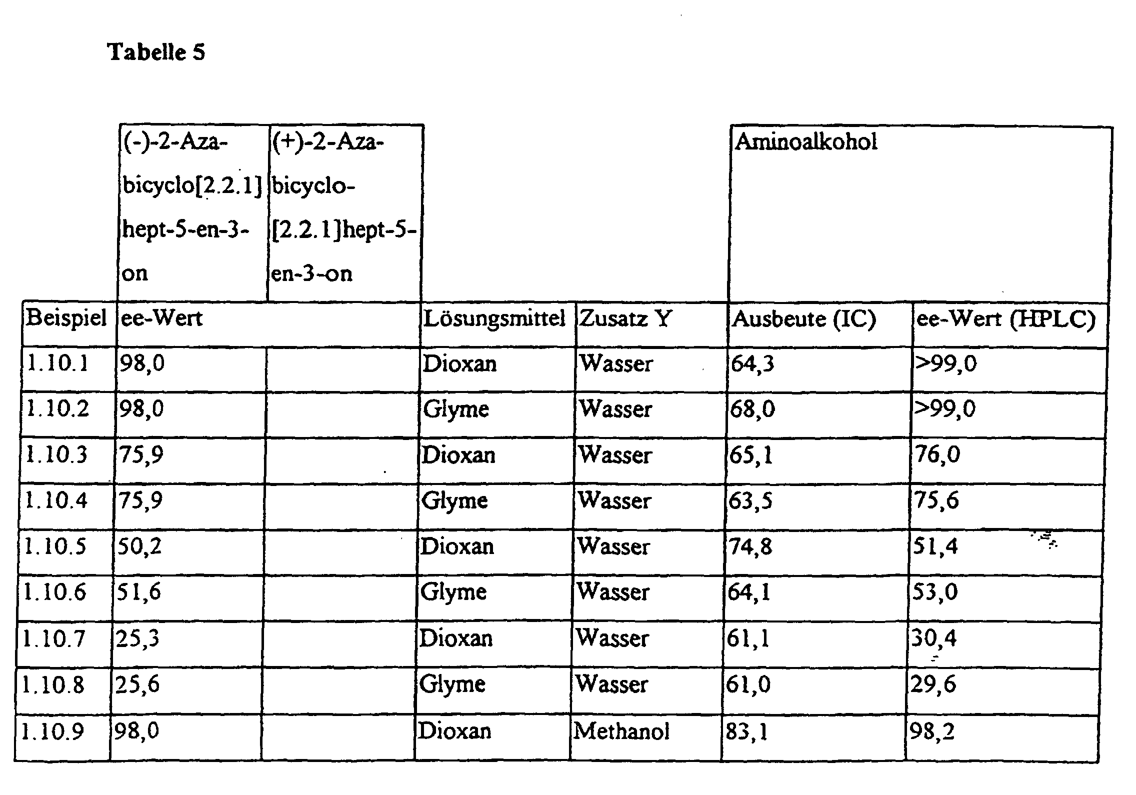

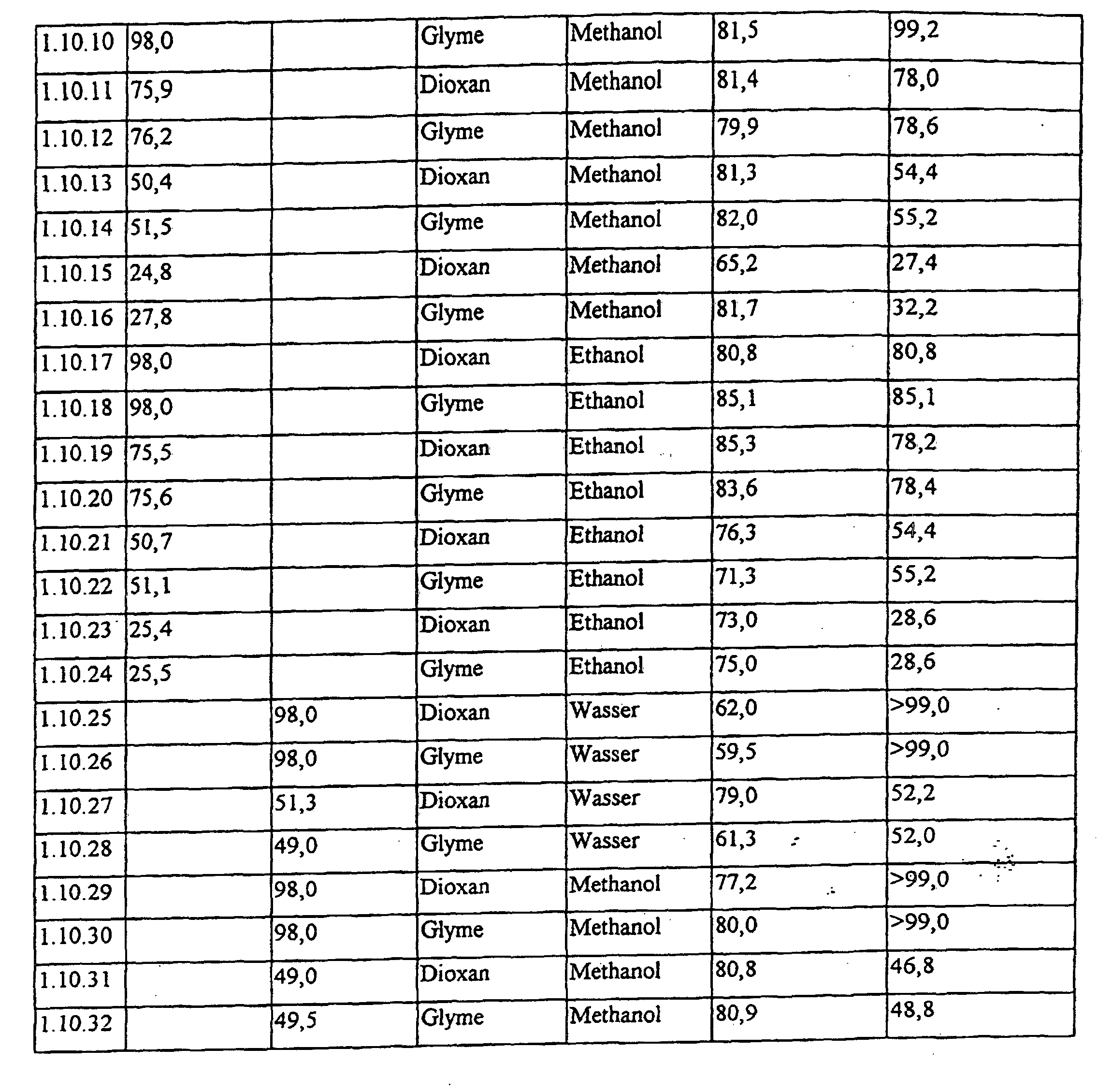

### 1.11 Herstellung von cis-(±)-1-Amino-4-(hydroxymethyl)-2-cyclopenten mittels Natriumborhydrid in verschiedenen Alkoholen

Entsprechend zu Beispiel 1.6 wurde die Reaktion in verschiedenen Zusätzen (Alkohole oder Wasser) durchgeführt. Im Unterschied zu Beispiel 1.6 wurde jedoch Natriumborhydrid (0,51g; 13,7 mmol) als Reduktionsmittel eingesetzt. Die Ergebinsse sind in Tabelle 6 zusammengefasst.

**Tabelle 6**

| Beispiel | Zusatz Y | X | X | (±)-2-Azabicyclo[2.2.1] hept-5-en-3-on | Aminoalkohol |
|---|---|---|---|---|---|
| | | mmol | Aequivalente | Ausbeute % | |
| 1.11.1 | Wasser | 17,1 | 1,25 | 75,4 | 20,1 |
| 1.11.2 | Wasser | 34,3 | 2,5 | 71,9 | 26,7 |
| 1.11.3 | Methanol | 34,3 | 2,5 | 39,2 | 22,2 |
| 1.11.4 | Ethanol | 34,3 | 2,5 | 67,8 | 8,6 |
| 1.11.5 | - | - | - | 62,2 | 3,5 |

### 1.12 Herstellung von cis-(±)-1-Amino-4-(hydroxymethyl)-2-cyclopenten mit NaBH₃CN

In einem 100 ml Sulfierkolben mit mechanischer Rührung wurden 60 ml Dioxan und 8,6 g (137 mmol) Natriumcyanborhydrid sowie 11,9 g (137 mmol) Lithiumbromid über Nacht für 15 h bei 110°C rückflussiert. Dann wurde auf 60°C abgekühlt und eine Lösung von 5,0 g (45,8 mmol) (±)-2-Azabicyclo[2.2.1]hept-5-en-3-on mit 15 ml Methanol während 30 min. zugetropft. Die weisse Suspension wurde für 3 h bei 60°C gerührt, abgekühlt auf ca. 5°C und auf ca. 100 ml halbkonzentrierte HCl gegeben. Der Gehalt wurde dann direkt durch ein quantitatives ionenchromatografisches Verfahren bestimmt. Die Ausbeute an Aminoalkohol betrug ca. 4%.

### Beispiel 2: Herstellung von (1R,4S)-Amino-4-(hydroxymethyl)-2-cyclopentenhydrochlorid

### 2.1 Reduktion von (-)-2-Azabicyclo[2.2.1]hept-5-en-3-on

In einen mit N₂ inertisierten 2 l - Autoklaven (V4A) wurden 61,4 g Natriumborhydrid 97,5% (1,623 Mol), 70,2 g Lithiumchlorid 98,5% (1,656 Mol), 13,2 g Celite sowie 1410 g Tetrahydrofuran eingefüllt. Der Autoklav wurde verschlossen, es wurde auf eine Innentemperatur von 130 °C aufgeheizt und 4,5 Stunden bei dieser Temperatur gerührt (max. 8,0 bar).

Nach Abkühlen auf ca. 60 °C wurden die in Tetrahydrofuran unlöslichen Natriumsalze (NaCl, NaBH₄) abfiltriert. Diese wurden mit 353 g Tetrahydrofuran gewaschen und die vereinigten Filtrate wurden in einem 1 l-Glasrührwerk durch Destillation bei Normaldruck auf ca. die Hälfte eingeengt (Destillat 1: ca. 710 g Tetrahydrofuran). Anschliessend wurde durch weitere Destillation, im Wechsel mit portionsweiser Zugabe von insgesamt 936 g Dioxan der Lösungsmitteltausch vollendet (Destillat 2: ca. 1289 g Tetrahydrofuran/Dioxan).
Zur auf ca. 60 °C gekühlten LiBH₄-Suspension wurden 56,7 g (-)-2-Azabicyclo[2.2.1]hept-5-en-3-on (97,5%) addiert. Bei ca. 60 °C beginnend wurden in exakt einer Stunde 132,5 g Methanol so zudosiert, dass ein Temperaturbereich von 58 - 62 °C eingehalten wurde. Es wurde eine weitere Stunde bei 60 °C nachreagieren gelassen. Anschliessend wurden weitere 397,0 g Methanol addiert (Probe enthält eine analytische Ausbeute von 70,5%) und der Rührwerksinhalt wurde auf 0 °C abgekühlt. Bei dieser Temperatur wurden 90,0 g HCl in die Reaktionsmischung eingeleitet (leicht exotherm) und es wurde eine weitere Stunde bei ca 0 °C gerührt. Durch Destillation bei Normaldruck (bis zu einer Kopftemperatur von 75 °C) wurden die Leichtsieder (Methanol, Borester) und ca. 70% des Dioxans entfernt (Destillat 3: ca. 1093 g). Anschliessend wurde durch Vakuumdestillation (ca. 30 mbar), im Wechsel mit portionsweiser Zugabe von insgesamt 282 g 1-Pentanol der Lösungsmitteltausch vollendet (Destillat 4: ca. 240 g Dioxan / Pentanol). Nach Zugabe weiterer 302 g 1-Pentanol wurde 1 Stunde bei 50 °C gerührt und die ausgefallenen Salze, ca. 39 g Feuchtgewicht, wurden abfiltriert und mit 200 g 1-Pentanol gewaschen. Die vereinigten Filtrate wurden durch erneute Vakuumdestillation (ca. 20 mbar) eingeengt (Destillat 5: ca. 235 g 1-Pentanol). Dann wurden bei ca. 50 °C 236 g Aceton zudosiert und die Reaktionsmischung wurde mit einigen Kristallen (1R,4S)-Amino-4-(hydroxymethyl)-2-cyclopenten angeimpft. Innerhalb einer Stunde wurde auf 5 °C abgekühlt und zur Vollendung der Kristallisation wurde weitere 6 h bei 5 °C gerührt.

Das Kristallisat wurde abfiltriert, mit 63 g Aceton gewaschen und bei max. 50 °C im Vakuumtrockenschrank (10 mbar) getrocknet. Man erhielt 83,5 g Rohprodukt * (Gehalt: 56,5%).
Dies entsprach einer Ausbeute von 61,4 % bzgl. eingesetztem (-)-2-Azabicyclo-[2.2. 1]hept-5-en-3-on.

### 2.2 Reduktion von (±)-2-Azabicyclo[2.2.1]hept-5-en-3-on

In einen mit N₂ inertisierten 2 l - Autoklaven (V4A) wurden 41,56 g Natriumborhydrid 97,5% (1,071 Mol), 51,48 g Lithiumchlorid 98,5% (1,196 Mol), 9,30 g Celite sowie 955,0 g Tetrahydrofuran eingefüllt. Der Autoklav wurde verschlossen, es wurde auf eine Innentemperatur von 130 °C aufgeheizt und 6 Stunden bei dieser Temperatur gerührt eine (max. 6,3 bar).
Nach Abkühlen auf ca. 60 °C wurden die in Tetrahydrofuran unlöslichen Natriumsalze (NaCl, NaBH₄) abfiltriert. Diese wurden mit 239,0 g Tetrahydrofuran gewaschen und die vereinigten Filtrate wurden in einem 1 l-Glasrührwerk durch Destillation bei Normaldruck auf ca die Hälfte eingeengt (Destillat 1: ca. 590 g THF).
Anschliessend wurde durch weitere Destillation, im Wechsel mit portionsweiser Zugabe von insgesamt 661,0 g Dioxan der Lösungsmitteltausch vollendet (Destillat 2: ca. 685 g Tetrahydrofuran/Dioxan). Zur aufca. 60 °C gekühlten LiBH₄-Suspension wurden 36,0 g 2-Azabicyclo[2.2.1]hept-5-en-3-on (97,5%) addiert. Bei ca. 60 °C beginnend wurden in exakt einer Stunde 77,6 g Methanol so zudosiert, dass ein Temperaturbereich von 58 - 62 °C eingehalten wurde. Es wurde eine weitere Stunde bei 60 °C nachreagieren gelassen. Anschliessend wurden weitere 233,0 g Methanol addiert und der Rührwerksinhalt wurde auf 0 °C abgekühlt. Bei dieser Temperatur wurden 52,9 g HCl in die Reaktionsmischung eingeleitet (leicht exotherm) und es wurde eine weitere Stunde bei ca 0 °C gerührt.
Durch Destillation bei Normaldruck (bis zu einer Kopftemperatur von 75 °C) wurden die Leichtsieder (Methanol, Borester) und ca. 70% des Dioxans entfernt (Destillat 3: ca. 700 g).

Anschliessend wurde durch Vakuumdestillation (ca. 30 mbar), im Wechsel mit portionsweiser Zugabe von insgesamt 169,4 g 1-Pentanol der Lösungsmitteltausch vollendet (Destillat 4: ca. 183 g Dioxan / Pentanol). Nach Zugabe weiterer 127,1 g 1-Pentanol wurde 1 Stunde bei 50 °C gerührt und die ausgefallenen Salze, ca.41 g Feuchtgewicht, wurden abfiltriert und mit 63,5 g 1-Pentanol gewaschen. Die vereinigten Filtrate wurden durch erneute Vakuumdestillation (ca. 20 mbar) eingeengt (Destillat 5: ca. 235 g 1-Pentanol). Dann wurden bei ca. 50 °C 238,0 g Aceton zudosiert und die Reaktionsmischung wurde mit einigen Kristallen Aminoalkohol-Hydrochloridsalz angeimpft. Innerhalb einer Stunde wurde auf 5 °C abgekühlt und zur Vollendung der Kristallisation wurde weitere 6 Stunden bei 5 °C gerührt.
Das Kristallisat wurde abfiltriert, mit 61,0 g Aceton gewaschen und bei max. 50 °C im Vakuumtrockenschrank (10 mbar) getrocknet. Man erhielt 50,0 g Rohprodukt (Gehalt: ca. 50% Aminoalkohol-Hydrochloridsalz). Dies entsprach einer Ausbeute von 52,0 % bzgl. eingesetztem 2-Azabicyclo[2.2.1]hept-5-en-3-on.

## Patentansprüche

1. Verfahren zur Herstellung eines Aminoalkohols der Formel 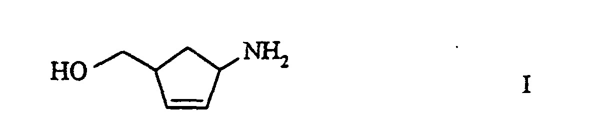 in Form des Racemats oder eines seiner optisch aktiven Isomeren, oder einem Salz davon, umfassend die Reduktion von 2-Azabicyclo[2.2.1]-hept-5-en-3-on der Formel 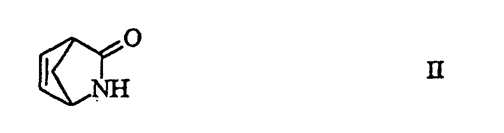 in Form des Racemats oder eines seiner optisch aktiven Isomeren mit einem Metallhydrid.

2. Verfahren nach Anspruch 1, **dadurch gekennzeichnet, dass** man als Metallhydrid ein Metallborhydrid einsetzt.

3. Verfahren nach Anspruch 1 oder 2, **dadurch gekennzeichnet, dass** man die Reduktion bei einer Temperatur von -20 bis 200 °C durchführt.

4. Verfahren nach einem der Ansprüche 1 bis 3, **dadurch gekennzeichnet, dass** man die Reduktion in einem aprotischen oder protischen organischen Lösungsmittel bzw. in einem entsprechenden Lösungsmittelgemisch durchführt.

5. Verfahren nach einem der Ansprüche 1 bis 4, **dadurch gekennzeichnet, dass** man die Reduktion in Gegenwart eines Zusatzes wie Wasser oder eines ein- oder mehrwertigen C₁₋₆-Alkohols durchführt.

## Claims

1. Process for the preparation of an aminoalcohol of the formula 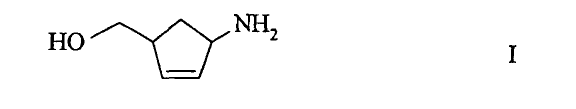 in the form of the racemate or one of its optically active isomers, or a salt thereof, comprising the reduction of2-azabicyclo-[2.2.1]hept-5-en-3-one of the formula 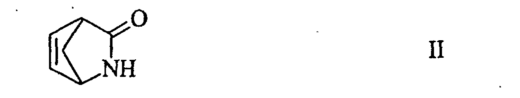 in the form of the racemate or one of its optically active isomers with a metal hydride.

2. Process according to Claim 1, **characterized in that** the metal hydride used is a metal borohydride.

3. Process according to Claim 1 or 2, **characterized in that** the reduction is carried out at a temperature of from -20 to 200 °C.

4. Process according to at least one of Claims 1 to 3, **characterized in that** the reduction is carried out in an aprotic or protic organic solvent or in a corresponding solvent mixture.

5. Process according to at least one of Claims 1 to 4, **characterized in that** the reduction is carried out in the presence of water or a mono- or polyhydric C₁₋₆-alcohol.

## Revendications

1. Procédé pour la préparation d'un aminoalcool de formule 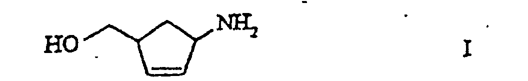 sous forme du racémate ou d'un de ses isomères optiquement actifs ou un sel de celui-ci, comprenant la réduction de la 2-azabicyclo[2.2.1]-hept-5-én-3-one de formule 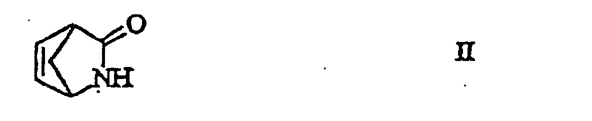 sous forme du racémate ou d'un de ses isomères optiquement actifs avec un hydrure de métal.

2. Procédé selon la revendication 1, **caractérisé en ce qu'**on utilise un borohydrure de métal comme hydrure de métal.

3. Procédé selon la revendication 1 ou 2, **caractérisé en ce qu'**on réalise la réduction à une température de -20 à 200°C.

4. Procédé selon l'une quelconque des revendications 1 à 3, **caractérisé en ce qu'**on réalise la réduction dans un solvant organique aprotique ou protique ou, selon le cas, dans un mélange de solvants correspondant.

5. Procédé selon l'une quelconque des revendications 1 à 4, **caractérisé en ce qu'**on réalise la réduction en présence d'un additif tel que de l'eau ou un alcool en C₁ à C₆ monovalent ou polyvalent.
